# EUROPEAN PATENT APPLICATION

(11) **EP 1 760 152 A1**
(43) Date of publication of application: **07.03.2007**
(21) Application number: 05734655.3
(22) Date of filing: 21.04.2005
(51) Int. Cl.: C12N 15/52, C12N 1/21, C12N 9/00, C12P 21/02

(54) **PROCESS FOR PRODUCING DIPEPTIDE**

(30) Priority: 21.04.2004 JP 2004125486
(71) Applicant: KYOWA HAKKO KOGYO CO., LTD., Chiyoda-ku, Tokyo 100-8185 (JP)
(72) Inventor: HASHIMOTO, Shin-ichi c/o Technical res. lab, Yamaguchi 747-8522 (JP); TABATA, Kazuhiko c/o BioFrontier Lab., Tokyo 194-8533 (JP); NOGUCHI, Ayako c/o Head Office, Kyowa Hakko Kogyo, Tokyo 100-8185 (JP); ADACHI, Yugo c/o Technical res. lab., Yamaguchi 747-8522 (JP)
(74) Representative: Vossius & Partner
(86) International application number: PCT/JP2005/007626
(87) International publication number: WO 2005/103260

(57) **Abstract**

The present invention provides: a protein having dipeptide-synthesizing activity or a protein for dipeptide synthesis; DNA encoding the protein having dipeptide-synthesizing activity or the protein for dipeptide synthesis; a recombinant DNA comprising the DNA; a transformant carrying the recombinant DNA; a process for producing the protein having dipeptide-synthesizing activity; an enzymatic process for producing a dipeptide using the protein having dipeptide-synthesizing activity or the protein for dipeptide synthesis; and a process for producing a dipeptide using, as an enzyme source, a culture of a microorganism or a transformant having the ability to produce the protein having dipeptide-synthesizing activity or the protein for dipeptide synthesis, or the like.

## Description

### Technical Field

The present invention relates to a protein having dipeptide-synthesizing activity or a protein for dipeptide synthesis, a process for producing the protein having dipeptide-synthesizing activity, a process for producing a dipeptide using the protein having dipeptide-synthesizing activity or the protein for dipeptide synthesis, a microorganism or a transformant which produces the protein having dipeptide-synthesizing activity or the protein for dipeptide synthesis, and a process for producing a dipeptide using the microorganism or the transformant.

### Background Art

As for the method for large-scale peptide synthesis, chemical synthesis methods (liquid phase method and solid phase method), enzymatic synthesis methods and biological synthesis methods utilizing recombinant DNA techniques are known. Currently, the enzymatic synthesis methods and biological synthesis methods are employed for the synthesis of long-chain peptides longer than 50 residues, and the chemical synthesis methods and enzymatic synthesis methods are mainly employed for the synthesis of dipeptides.

In the synthesis of dipeptides by the chemical synthesis methods, operations such as introduction and removal of protective groups for functional groups are necessary, and racemates are also formed. The chemical synthesis methods are thus considered to be disadvantageous in respect of cost and efficiency. They are unfavorable also from the viewpoint of environmental hygiene because of the use of large amounts of organic solvents and the like.

As to the synthesis of dipeptides by the enzymatic methods, the following methods are known: a method utilizing reverse reaction of protease [J. Biol. Chem., 119, 707-720 (1937)]; methods utilizing thermostable aminoacyl t-RNA synthetase (Japanese Published Unexamined Patent Application No. 146539/83, Japanese Published Unexamined Patent Application No. 209991/83, Japanese Published Unexamined Patent Application No. 209992/83 and Japanese Published Unexamined Patent Application No. 106298/84); and methods utilizing non-ribosomal peptide synthetase (hereinafter referred to as NRPS) [Chem. Biol., 7, 373-384 (2000); FEBS Lett., 498, 42-45 (2001); US Patent No. 5795738; US Patent No. 5652116].

However, the method utilizing reverse reaction of protease requires introduction and removal of protective groups for functional groups of amino acids used as substrates, which causes difficulties in raising the efficiency of peptide-forming reaction and in preventing peptidolytic reaction. The methods utilizing thermostable aminoacyl t-RNA synthetase have the defects that the expression of the enzyme and the prevention of side reactions forming by-products other than the desired products are difficult. The methods utilizing NRPS are inefficient in that the expression of the enzyme by recombinant DNA techniques is difficult because the enzyme molecule is huge, and in that the supply of coenzyme 4'-phosphopantetheine is necessary.

On the other hand, there exist a group of peptide synthetases that have enzyme molecular weight lower than that of NRPS and do not require coenzyme 4'-phosphopantetheine; for example, γ-glutamylcysteine synthetase, glutathione synthetase, D-alanine-D-alanine (D-Ala-D-Ala) ligase, and poly-γ-glutamate synthetase. Most of these enzymes utilize D-amino acids as substrates or catalyze peptide bond formation at the γ-carboxyl group. Because of such properties, they can not be used for the synthesis of dipeptides by peptide bond formation at the α-carboxyl group of L-amino acid.

The only known example of an enzyme capable of forming a dipeptide by the activity to form a peptide bond at the α-carboxyl group of L-amino acid is bacilysin (dipeptide antibiotic derived from a microorganism belonging to the genus Bacillus) synthetase. Bacilysin synthetase is known to have the activity to synthesize bacilysin [L-alanyl-L-anticapsin (L-Ala-L-anticapsin)] and L-alanyl-L-alanine (L-Ala-L-Ala), but there is no information about its activity to synthesize other dipeptides [J. Ind. Microbiol., 2, 201-208 (1987); Enzyme Microbial. Technol., 29, 400-406 (2001)].

Certain microorganisms are known to form a compound having the cyclic dipeptide (diketopiperazine) structure wherein two amino acids are cyclically bound [J. Nat. Prod., 59, 293-296 (1996); Tetrahedron, 28, 2999 (1972); J. Appl. Microbiol., 86, 29-53 (1999)]. With regard to the biosynthesis of diketopiperazine, it is reported that the cyclo-(L-4-nitrotryptophyl-L-phenylalanine) structure is synthesized by NRPS in the Thaxtomin biosynthesis process of Streptomyces acidiscabies [Mol. Microbiol., 38, 794-804 (2000)] and that cyclo(phenylalanyl-proline) is formed from phenylalanine and proline by the action of a part of the modules of NRPS of bacteria belonging to the genus Bacillus [J. Biol. Chem., 273, 22773-22781 (1998)].

It is also reported that a protein bearing no similarity to NRPS (albC gene product) is responsible for the synthesis of the cyclo(L-phenylalanyl-L-leucine) structure in Streptomyces noursei ATCC 11455 known as a strain producing the antibiotic albonoursin and that albonoursin was detected when cyclo dipeptide oxidase was made to act on the culture liquor of Escherichia coli and Streptomyces lividans into which the albC gene was introduced [Chemistry & Biol., 9, 1355-1364 (2002)]. However, there is no report that the albC gene product forms a straight-chain dipeptide.

### Disclosure of the Invention

### Problems to be Solved by the Invention

An object of the present invention is to provide: a protein having dipeptide-synthesizing activity or a protein for dipeptide synthesis; DNA encoding the protein having dipeptide-synthesizing activity or the protein for dipeptide synthesis; a recombinant DNA comprising the DNA; a transformant carrying the recombinant DNA; a process for producing the protein having dipeptide-synthesizing activity; an enzymatic process for synthesizing a dipeptide using the protein having dipeptide-synthesizing activity or the protein for dipeptide synthesis; and a process for producing a dipeptide using, as an enzyme source, a culture of a microorganism or a transformant having the ability to produce the protein having dipeptide-synthesizing activity or the protein for dipeptide synthesis, or the like.

### Means for Solving the Problems

The present invention relates to the following (1) to (24).
(1) A protein according to any of the following [1] to [3], provided that a protein consisting of the amino acid sequence shown in SEQ ID NO: 1 is excluded:
   [1] a protein having the amino acid sequence shown in SEQ ID NO: 2;
   [2] a protein consisting of an amino acid sequence wherein one or more amino acid residues are deleted, substituted or added in the amino acid sequence shown in SEQ ID NO: 1 or 2 and having the activity to synthesize a dipeptide represented by formula (I):

      R¹ - R² (I)

      (wherein R¹ and R², which may be the same or different, each represent an amino acid); and
   [3] a protein consisting of an amino acid sequence which has 65% or more homology to the amino acid sequence shown in SEQ ID NO: 1 or 2 and having the activity to synthesize a dipeptide represented by formula (I).
(2) A protein for dipeptide synthesis according to any of the following [1] to [3]:
   [1] a protein for dipeptide synthesis having the amino acid sequence shown in SEQ ID NO: 1;
   [2] a protein for dipeptide synthesis consisting of an amino acid sequence wherein one or more amino acid residues are deleted, substituted or added in the amino acid sequence shown in SEQ ID NO: 1 and having the activity to synthesize a dipeptide represented by formula (I):

      R¹- R² (I)

      (wherein R¹ and R², which may be the same or different, each represent an amino acid); and
   [3] a protein for dipeptide synthesis consisting of an amino acid sequence which has 65% or more homology to the amino acid sequence shown in SEQ ID NO: 1 and having the activity to synthesize a dipeptide represented by formula (I).
(3) A DNA according to any of the following [1] to [3], provided that a DNA consisting of the nucleotide sequence shown in SEQ ID NO: 3 is excluded:
   [1] DNA encoding the protein according to the above (1);
   [2] DNA having the nucleotide sequence shown in SEQ ID NO: 4; and
   [3] DNA which hybridizes with DNA having a nucleotide sequence complementary to the nucleotide sequence shown in SEQ ID NO: 4 under stringent conditions and which encodes a protein having the activity to synthesize a dipeptide represented by formula (I):

      R¹- R² (I)

      (wherein R¹ and R², which may be the same or different, each represent an amino acid).
(4) A recombinant DNA comprising the DNA according to the above (3).
(5) A transformant carrying the recombinant DNA according to the above (4).
(6) The transformant according to the above (5), wherein the transformant is a transformant obtainable by using a microorganism as a host.
(7) The transformant according to the above (6), wherein the microorganism is a microorganism belonging to the genus Escherichia.
(8) A process for producing the protein according to the above (1), which comprises culturing the transformant according to any of the above (5) to (7) in a medium, allowing the protein according to the above (1) to form and accumulate in the culture, and recovering the protein from the culture.
(9) A process for producing the protein according to the above (1), which comprises culturing a microorganism having the ability to produce the protein according to the above (1) in a medium, allowing the protein to form and accumulate in the culture, and recovering the protein from the culture.
(10) The process according to the above (9), wherein the microorganism is a microorganism belonging to the genus Streptomyces.
(11) The process according to the above (10), wherein the microorganism belonging to the genus Streptomyces is a microorganism belonging to the genus Streptomyces which has the ability to produce albonoursin.
(12) The process according to the above (11), wherein the microorganism belonging to the genus Streptomyces which has the ability to produce albonoursin is
   Streptomyces albulus or Streptomyces noursei.
(13) A process for producing a dipeptide represented by formula (I):

   R¹ - R² (I)

   (wherein R¹ and R², which may be the same or different, each represent an amino acid), which comprises:
   allowing the protein according to the above (1) or the protein for dipeptide synthesis according to the above (2), one or more kinds of amino acids, and ATP to be present in an aqueous medium;
   allowing the dipeptide to form and accumulate in the medium; and
   recovering the dipeptide from the medium.
(14) A process for producing a dipeptide represented by formula (I):

   R¹ - R² (I)

   (wherein R¹ and R², which may be the same or different, each represent an amino acid), which comprises:
   allowing an enzyme source and one or more kinds of amino acids to be present in an aqueous medium, said enzyme source being a culture or a treated matter of the culture selected from the group consisting of the following [1] to [3]:
      [1] a culture of the transformant according to any of the above (5) to (7) or a treated matter of the culture;
      [2] a culture of a microorganism having the ability to produce the protein according to the above (1) or a treated matter of the culture; and
      [3] a culture of a microorganism having the ability to produce the protein for dipeptide synthesis according to the above (2) or a treated matter of the culture;
      allowing the dipeptide to form and accumulate in the medium; and
      recovering the dipeptide from the medium.
(15) The process according to the above (14), wherein the microorganism having the ability to produce the protein according to the above (1) is a microorganism belonging to the genus Streptomyces.
(16) The process according to the above (14), wherein the microorganism having the ability to produce the protein for dipeptide synthesis according to the above (2) is a microorganism belonging to the genus Streptomyces.
(17) The process according to the above (15) or (16), wherein the microorganism belonging to the genus Streptomyces is a microorganism belonging to the genus Streptomyces which has the ability to produce albonoursin.
(18) The process according to the above (17), wherein the microorganism belonging to the genus Streptomyces which has the ability to produce albonoursin is a microorganism belonging to Streptomyces albulus or Streptomyces noursei.
(19) The process according to the above (14), wherein the microorganism having the ability to produce the protein for dipeptide synthesis according to the above (2) is a microorganism transformed with DNA encoding the protein for dipeptide synthesis according to the above (2).
(20) The process according to the above (19), wherein the microorganism transformed with DNA encoding the protein for dipeptide synthesis according to the above (2) is a microorganism belonging to the genus Escherichia.
(21) The process according to any of the above (14) to (20), wherein the treated matter of the culture is concentrated culture, dried culture, cells obtainable by centrifuging the culture, dried cells, freeze-dried cells, surfactant-treated cells, ultrasonic-treated cells, mechanically disrupted cells, solvent-treated cells, enzyme-treated cells, protein fractionation of the cells, immobilized cells, or an enzyme preparation obtainable from the cells by extraction.
(22) The process according to any of the above (13) to (21), wherein the one or more kinds of amino acids are L- or D-form of amino acids, glycine, β-alanine or derivatives thereof.
(23) The process according to any of the above (13) to (22), wherein the dipeptide is a dipeptide represented by formula (II):

   R³ - R⁴ (II)

   (wherein R³ and R⁴, which may be the same or different, each represent L- or D-form of amino acid, glycine, β-alanine or a derivative thereof).
(24) The process according to the above (22) or (23), wherein the L- or D-form of amino acid is an amino acid selected from the group consisting of alanine, glutamine, glutamic acid, valine, leucine, isoleucine, proline, phenylalanine, tryptophan, methionine, serine, threonine, cysteine, asparagine, tyrosine, lysine, arginine, histidine, aspartic acid, α-aminobutyric acid, azaserine, theanine, 4-hydroxyproline, 3-hydroxyproline, ornithine, citrulline and 6-diazo-5-oxo-norleucine.

### Effect of the Invention

The present invention provides: a protein having dipeptide-synthesizing activity or a protein for dipeptide synthesis; DNA encoding the protein having dipeptide-synthesizing activity or the protein for dipeptide synthesis; a recombinant DNA comprising the DNA; a transformant carrying the recombinant DNA; a process for producing the protein having dipeptide-synthesizing activity; an enzymatic process for producing a dipeptide using the protein having dipeptide-synthesizing activity or the protein for dipeptide synthesis; and a process for producing a dipeptide using, as an enzyme source, a culture of a microorganism or a transformant having the ability to produce the protein having dipeptide-synthesizing activity or the protein for dipeptide synthesis, or the like.

### Brief Description of the Drawings

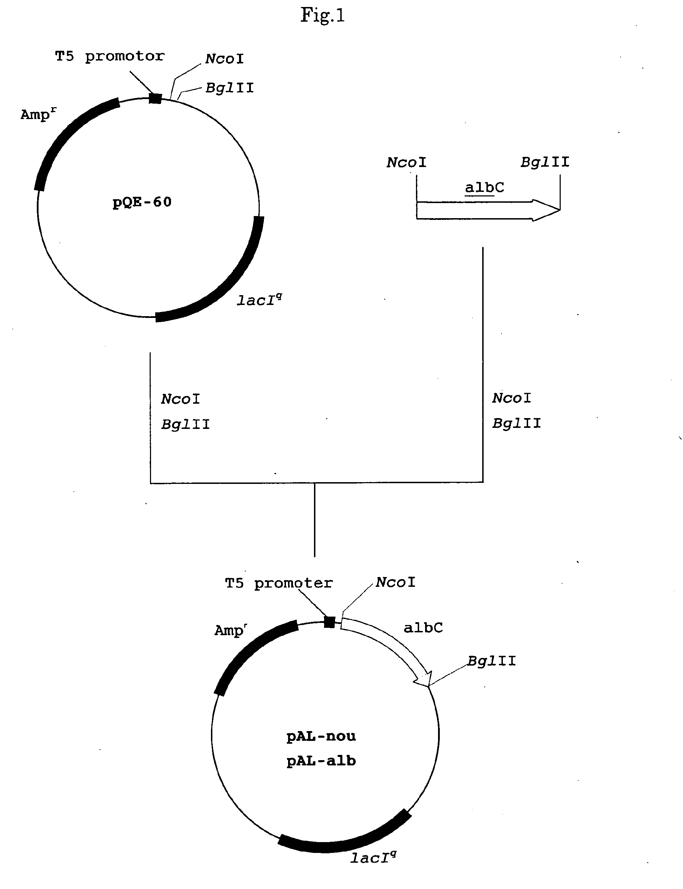

Fig. 1 shows the steps for constructing plasmid vectors pAL-nou and pAL-alb, which express a protein having dipeptide-synthesizing activity.

### Explanation of Symbols

Amp^{r}: Ampicillin resistance gene
lacI^{q}: Lactose repressor gene
albC: albC gene or albC-analogous gene

### Best Modes for Carrying Out the Invention

The proteins of the present invention include proteins of the following [1] to [3] (excluding a protein consisting of the amino acid sequence shown in SEQ ID NO: 1):
[1] a protein having the amino acid sequence shown in SEQ ID NO: 2;
[2] a protein consisting of an amino acid sequence wherein one or more amino acid residues are deleted, substituted or added in the amino acid sequence shown in SEQ ID NO: 1 or 2 and having the activity to synthesize a dipeptide represented by formula (I):

   R¹ - R² (I)

   (wherein R¹ and R², which may the same or different, each represent an amino acid); and
[3] a protein consisting of an amino acid sequence which has 65% or more homology, preferably 80% or more homology, more preferably 90% or more homology, further preferably 95% or more homology, particularly preferably 98% or more homology, most preferably 99% or more homology to the amino acid sequence shown in SEQ ID NO: 1 or 2 and having the activity to synthesize a dipeptide represented by formula (I).
   The proteins for dipeptide synthesis of the present invention include proteins of the following [4] to [6]:
[4] a protein for dipeptide synthesis having the amino acid sequence shown in SEQ ID NO: 1;
[5] a protein for dipeptide synthesis consisting of an amino acid sequence wherein one or more amino acid residues are deleted, substituted or added in the amino acid sequence shown in SEQ ID NO: 1 and having the activity to synthesize a dipeptide represented by formula (I); and
[6] a protein for dipeptide synthesis consisting of an amino acid sequence which has 65% or more homology, preferably 80% or more homology, more preferably 90% or more homology, further preferably 95% or more homology, particularly preferably 98% or more homology, most preferably 99% or more homology to the amino acid sequence shown in SEQ ID NO: 1 and having the activity to synthesize a dipeptide represented by formula (I).

Hereinafter, the above proteins and proteins for dipeptide synthesis of the present invention are sometimes referred to as the proteins of the present invention collectively.

The above protein consisting of an amino acid sequence wherein one or more amino acid residues are deleted, substituted or added and having the activity to synthesize a dipeptide represented by formula (I) can be obtained, for example, by introducing a site-directed mutation into DNA encoding a protein consisting of the amino acid sequence shown in SEQ ID NO: 1 or 2 by site-directed mutagenesis described in Molecular Cloning, A Laboratory Manual, Third Edition, Cold Spring Harbor Laboratory Press (1989) (hereinafter referred to as Molecular Cloning, Third Edition); Current Protocols in Molecular Biology, John Wiley & Sons (1987-1997) (hereinafter referred to as Current Protocols in Molecular Biology); Nucleic Acids Research, 10, 6487 (1982); Proc. Natl. Acad. Sci. USA, 79, 6409 (1982); Gene, 34, 315 (1985); Nucleic Acids Research, 13, 4431 (1985); Proc. Natl. Acad. Sci. USA, 82, 488 (1985), etc.

The number of amino acid residues which are deleted, substituted or added is not specifically limited, but is within the range where deletion, substitution or addition is possible by known methods such as the above site-directed mutagenesis. The suitable number is 1 to dozens, preferably 1 to 20, more preferably 1 to 10, further preferably 1 to 5.

The expression "one or more amino acid residues are deleted, substituted or added in the amino acid sequence shown in SEQ ID NO: 1 or 2" means that the amino acid sequence may contain deletion, substitution or addition of a single or plural amino acid residues at an arbitrary position therein.

Amino acid residues that may be substituted are, for example, those which are not conserved in all of the amino acid sequences shown in SEQ ID NOS: 1 and 2 when the sequences are compared using known alignment software. An example of known alignment software is alignment analysis software contained in gene analysis software Genetyx (Software Development Co., Ltd.). As analysis parameters for the analysis software, default values can be used.

Deletion or addition of amino acid residues may be contained, for example, in the N-terminal region or the C-terminal region of the amino acid sequence shown in SEQ ID NO: 1 or 2.

Deletion, substitution and addition may be simultaneously contained in one sequence, and amino acids to be substituted or added may be either natural or not. Examples of the natural amino acids are L-alanine, L-asparagine, L-aspartic acid, L-glutamine, L-glutamic acid, glycine, L-histidine, L-isoleucine, L-leucine, L-lysine, L-methionine, L-phenylalanine, L-proline, L-serine, L-threonine, L-tryptophan, L-tyrosine, L-valine and L-cysteine.

The following are examples of the amino acids capable of mutual substitution. The amino acids in the same group can be mutually substituted.
- Group A:: leucine, isoleucine, norleucine, valine, norvaline, alanine, 2-aminobutanoic acid, methionine, O-methylserine, t-butylglycine, t-butylalanine, cyclohexylalanine
- Group B:: aspartic acid, glutamic acid, isoaspartic acid, isoglutamic acid, 2-aminoadipic acid, 2-aminosuberic acid
- Group C:: asparagine, glutamine
- Group D:: lysine, arginine, ornithine, 2,4-diaminobutanoic acid, 2,3-diaminopropionic acid
- Group E:: proline, 3-hydroxyproline, 4-hydroxyproline
- Group F:: serine, threonine, homoserine
- Group G:: phenylalanine, tyrosine

In order that the protein of the present invention may have the activity to synthesize a dipeptide represented by formula (I), it is desirable that the homology of its amino acid sequence to the amino acid sequence shown in SEQ ID NO: 1 or 2 is 65% or more, preferably 80% or more, more preferably 90% or more, further preferably 95% or more, particularly preferably 98% or more, and most preferably 99% or more.

The homology among amino acid sequences and nucleotide sequences can be determined by using algorithm BLAST by Karlin and Altschul [Proc. Natl. Acad. Sci. USA, 90, 5873 (1993)] and FASTA [Methods Enzymol., 183, 63 (1990)]. On the basis of the algorithm BLAST, programs such as BLASTN and BLASTX have been developed [J. Mol. Biol., 215, 403 (1990)]. When a nucleotide sequence is analyzed by BLASTN on the basis of BLAST, the parameters, for instance, are as follows: score=100 and wordlength=12. When an amino acid sequence is analyzed by BLASTX on the basis of BLAST, the parameters, for instance, are as follows: score=50 and wordlength=3. When BLAST and Gapped BLAST programs are used, default parameters of each program are used. The specific techniques for these analyses are known (http://www.ncbi.nlm.nih.gov.).

It is possible to confirm that the protein of the present invention is a protein having the activity to synthesize a dipeptide represented by formula (I), for example, in the following manner. That is, a transformant expressing the protein of the present invention is prepared by recombinant DNA techniques, the protein of the present invention is produced using the transformant, and then the protein of the present invention, one or more kinds of amino acids and ATP are allowed to be present in an aqueous medium, followed by HPLC analysis or the like to know whether a dipeptide represented by the above formula (I) is formed and accumulated in the aqueous medium.

The DNAs of the present invention include DNAs of the following [1] to [3] (excluding DNA consisting of the nucleotide sequence shown in SEQ ID NO: 3):
[1] DNA encoding a protein having the amino acid sequence shown in SEQ ID NO: 2;
[2] DNA having the nucleotide sequence shown in SEQ ID NO: 4; and
[3] DNA which hybridizes with DNA having a nucleotide sequence complementary to the nucleotide sequence shown in SEQ ID NO: 4 under stringent conditions and which encodes a protein having the activity to synthesize a dipeptide represented by formula (I).
   The DNAs that can be used in the process for producing a dipeptide represented by formula (I) of the present invention include DNAs of the following [4] to [6] in addition to the DNAs of the above [1] to [3]:
[4] DNA encoding a protein having the amino acid sequence shown in SEQ ID NO: 1;
[5] DNA having the nucleotide sequence shown in SEQ ID NO: 3; and
[6] DNA which hybridizes with DNA having a nucleotide sequence complementary to the nucleotide sequence shown in SEQ ID NO: 3 under stringent conditions and which encodes a protein having the activity to synthesize a dipeptide represented by formula (I).

"To hybridize" refers to hybridization of DNA with DNA having a specific nucleotide sequence or a part of the DNA. Therefore, the nucleotide sequence of the DNA having a specific nucleotide sequence or a part of the DNA may be DNA which is long enough to be useful as a probe for Northern or Southern blot analysis or to be used as an oligonucleotide primer for PCR analysis. DNAs used as a probe include DNAs consisting of at least 100 nucleotides, preferably 200 or more nucleotides, more preferably 500 or more nucleotides, but may also be DNAs consisting of at least 10 nucleotides, preferably 15 or more nucleotides.

The method for hybridization of DNA is well known and the conditions for hybridization can be determined by a person skilled in the art according to the present specification. The hybridization can be carried out according to the methods described in Molecular Cloning, Second Edition, Third Edition (2001); Methods for General and Molecular Bacteriology, ASM Press (1994); Immunology methods manual, Academic press (Molecular), and many other standard textbooks.

Hybridization under the above stringent conditions is preferably carried out, for example, as follows. A filter with DNA immobilized thereon and a probe DNA are incubated in a solution comprising 50% formamide, 5 x SSC (750 mM sodium chloride and 75 mM sodium citrate), 50 mM sodium phosphate (pH 7.6), 5 x Denhardt's solution, 10% dextran sulfate and 20 µg/l denatured salmon sperm DNA at 42°C overnight, and after the incubation, the filter is washed in 0.2 x SSC solution (ca. 65°C). Lower stringent conditions can also be employed. Modification of the stringent conditions can be made by adjusting the concentration of formamide (the conditions become low stringent as the concentration of formamide is lowered) and by changing the salt concentrations and the temperature conditions. Hybridization under low stringent conditions is carried out, for example, by incubating a filter with DNA immobilized thereon and a probe DNA in a solution comprising 6 x SSCE (20 x SSCE: 3 mol/l sodium chloride, 0.2 mol/l sodium dihydrogenphosphate and 0.02 mol/l EDTA, pH 7.4), 0.5% SDS, 30% formamide and 100 µg/l denatured salmon sperm DNA at 37°C overnight, and washing the filter with 1 x SSC solution containing 0.1% SDS (50°C). Hybridization under still low stringent conditions is carried out by using a solution having a high salt concentration (for example, 5 x SSC) under the above low stringent conditions, followed by washing.

Various conditions described above can also be established by adding a blocking reagent used to reduce the background of hybridization or changing the reagent. The addition of the above blocking reagent may be accompanied by changes of conditions for hybridization to make the conditions suitable for the purpose.

The above DNA capable of hybridization under stringent conditions includes DNA having at least 90% homology, preferably 95% or more homology, more preferably 98% or more homology, further preferably 99% or more homology to the nucleotide sequence shown in SEQ ID NO: 3 or 4 as calculated by use of programs such as BLAST and FASTA described above based on the above parameters.

It is possible to confirm that the DNA which hybridizes with DNA having the nucleotide sequence shown in SEQ ID NO: 3 or 4 under stringent conditions is DNA encoding a protein having the activity to synthesize a dipeptide represented by formula (I), for example, by producing a protein encoded by the DNA by recombinant DNA techniques and measuring the activity of the protein as described above.

### (i) Preparation of the DNA of the Present Invention and DNA Used in the Process for Producing a Protein or a Dipeptide of the Present Invention

The DNA of the present invention and the DNA used in the process for producing a protein or a dipeptide of the present invention (hereinafter, also referred to as the production process of the present invention) can be obtained, for example, by Southern hybridization of a chromosomal DNA library derived from a microorganism belonging to the genus Streptomyces using a probe designed based on the nucleotide sequence shown in SEQ ID NO: 3 or 4, or by PCR [PCR Protocols, Academic Press (1990)] using primer DNAs designed based on the nucleotide sequence shown in SEQ ID NO: 3 or 4, and as a template, the chromosomal DNA of a microorganism belonging to the genus Streptomyces.

The DNA of the present invention and the DNA used in the production process of the present invention can also be obtained by conducting a search through various gene sequence databases for a sequence having 75% or more homology, preferably 85% or more homology, more preferably 90% or more homology, further preferably 95% or more homology, particularly preferably 98% or more homology, most preferably 99% or more homology to the nucleotide sequence of DNA encoding the amino acid sequence shown in SEQ ID NO: 1 or 2, and obtaining the desired DNA, based on the nucleotide sequence obtained by the search, from a chromosomal DNA or cDNA library of an organism having the nucleotide sequence according to the above-described method.

The obtained DNA, as such or after cleavage with appropriate restriction enzymes, is inserted into a vector by a conventional method, and the obtained recombinant DNA is introduced into a host cell. Then, the nucleotide sequence of the DNA can be determined by a conventional sequencing method such as the dideoxy method [Proc. Natl. Acad. Sci., USA, 74, 5463 (1977)] or by using a nucleotide sequencer such as 373A DNA Sequencer (Perkin-Elmer Corp.).

In cases where the obtained DNA is found to be a partial DNA by the analysis of nucleotide sequence, the full length DNA can be obtained by Southern hybridization of a chromosomal DNA library using the partial DNA as a probe.

It is also possible to prepare the desired DNA by chemical synthesis using a DNA synthesizer (e.g., Model 8905, PerSeptive Biosystems) based on the determined nucleotide sequence of the DNA.

Examples of the DNAs that can be obtained by the above-described method are DNAs having the nucleotide sequences shown in SEQ ID NOS: 3 and 4.

Examples of the vectors for inserting the DNA of the present invention or the DNA used in the production process of the present invention include pBluescript II KS(+) (Stratagene), pDIRECT [ Nucleic Acids Res., 18, 6069 (1990)], pCR-Script Amp SK(+) (Stratagene), pT7Blue (Novagen, Inc.), pCR II (Invitrogen Corp.) and pCR-TRAP (Genhunter Corp.).

As the host cell, microorganisms belonging to the genus Escherichia, etc. can be used. Examples of the microorganisms belonging to the genus Escherichia include Escherichia coli XL1-Blue, Escherichia coli XL2-Blue, Escherichia coli DH1, Escherichia coli MC1000, Escherichia coli KY3276, Escherichia coli W1485, Escherichia coli JM109, Escherichia coli HB101, Escherichia coli No. 49, Escherichia coli W3110, Escherichia coli NY49, Escherichia coli MP347, Escherichia coli NM522 and Escherichia coli ME8415.

Introduction of the recombinant DNA can be carried out by any of the methods for introducing DNA into the above host cells, for example, the method using calcium ion [Proc. Natl. Acad. Sci. USA, 69, 2110 (1972)], the protoplast method (Japanese Published Unexamined Patent Application No. 248394/88) and electroporation [Nucleic Acids Res., 16, 6127 (1988)].

An example of the microorganism carrying the DNA used in the production process of the present invention obtained by the above method is Escherichia coli NM522/pAL-nou, which is a microorganism carrying a recombinant DNA comprising DNA having the sequence shown in SEQ ID NO: 3.

### (ii) Process for Producing the Protein and the Protein for Dipeptide Synthesis of the Present Invention

The protein and the protein for dipeptide synthesis of the present invention can be produced by expressing the DNA of the present invention or the DNA used in the production process of the present invention obtained by the methods of the above (i) in host cells using the methods described in Molecular Cloning, Third Edition, Current Protocols in Molecular Biology, etc., for example, in the following manner.

On the basis of the DNA of the present invention or the DNA used in the production process of the present invention, a DNA fragment of an appropriate length comprising a region encoding the protein or the protein for dipeptide synthesis of the present invention is prepared according to need. The productivity of the protein can be enhanced by replacing a nucleotide in the nucleotide sequence of the region encoding the protein so as to make a codon most suitable for the expression in a host cell.

The DNA fragment is inserted downstream of a promoter in an appropriate expression vector to prepare a recombinant DNA.

A transformant which produces the protein of the present invention can be obtained by introducing the recombinant DNA into a host cell suited for the expression vector.

As the host cell, any bacteria cells, yeast cells, animal cells, insect cells, plant cells, etc. that are capable of expressing the desired gene can be used. Preferred are bacterial cells, more preferred are microorganisms belonging to the genera Escherichia, Bacillus and Corynebacterium, and further preferred are microorganisms belonging to the genus Escherichia.

The expression vectors that can be employed are those capable of autonomous replication or integration into the chromosome in the above host cells and comprising a promoter at a position appropriate for the transcription of the DNA of the present invention or the DNA used in the production process of the present invention.

When a procaryote such as a bacterium is used as the host cell, it is preferred that the recombinant DNA comprising the DNA of the present invention or the DNA used in the production process of the present invention is a recombinant DNA which is capable of autonomous replication in the procaryote and which comprises a promoter, a ribosome binding sequence, the DNA of the present invention or the DNA used in the production process of the present invention, and a transcription termination sequence. The recombinant DNA may further comprise a gene regulating the promoter.

Examples of suitable expression vectors are pBTrp2, pBTac1 and pBTac2 (products of Boehringer Mannheim GmbH), pHelix1 (Roche Diagnostics Corp.), pKK233-2 (Amersham Pharmacia Biotech), pSE280 (Invitrogen Corp.), pGEMEX-1 (Promega Corp.), pQE8 (Qiagen, Inc.), pQE60 (Qiagen, Inc.), pET-3 (Novagen, Inc.), pKYP10 (Japanese Published Unexamined Patent Application No. 110600/83), pKYP200 [Agric. Biol. Chem., 48, 669 (1984)], pLSA1 [Agric. Biol. Chem., 53, 277 (1989)], pGEL1 [Proc. Natl. Acad. Sci. USA, 82, 4306 (1985)], pBluescript II SK(+), pBluescript II KS(-) (Stratagene), pTrS30 [prepared from Escherichia coli JM109/pTrS30 (FERM BP-5407)], pTrS32 [prepared from Escherichia coli JM109/pTrS32 (FERM BP-5408)], pPAC31 (WO98/12343), pUC19 [Gene, 33, 103 (1985)], pSTV28 (Takara Shuzo Co., Ltd.), pUC118 (Takara Shuzo Co., Ltd.) and pPA1 (Japanese Published Unexamined Patent Application No. 233798/88).

As the promoter, any promoters capable of functioning in host cells such as Escherichia coli can be used. For example, promoters derived from Escherichia coli or phage, such as trp promoter (Pₜᵣₚ), lac promoter (P_{lac}) , P_{L} promoter, P_{R} promoter and P_{SE} promoter, SP01 promoter, SPO2 promoter and penP promoter can be used. Artificially designed and modified promoters such as a promoter in which two PₜᵣₚS are combined in tandem, tac promoter, lacT7 promoter and letI promoter, etc. can also be used.

Also useful are promoters such as xylA promoter for the expression in microorganisms belonging to the genus Bacillus [ Appl. Microbiol. Biotechnol., 35, 594-599 (1991)], P54-6 promoter for the expression in microorganisms belonging to the genus Corynebacterium [Appl. Microbiol. Biotechnol., 53, 674-679 (2000)], and xylA promoter for the expression in microorganisms belonging to the genus Streptomyces (Genetic Manipulation of Streptomyces: a Laboratory Manual: John Innes Foundation).

It is preferred to use a plasmid in which the distance between the Shine-Dalgarno sequence (ribosome binding sequence) and the initiation codon is adjusted to an appropriate length (e.g., 6 to 18 nucleotides).

In the recombinant DNA wherein the DNA of the present invention or the DNA used in the production process of the present invention is ligated to an expression vector, the transcription termination sequence is not essential, but it is preferred to place the transcription termination sequence immediately downstream of the structural gene.

Examples of such recombinant DNAs are pAL-nuo and pAL-alb.

Examples of procaryotes include microorganisms belonging to the genera Escherichia, Serratia, Bacillus, Brevibacterium, Corynebacterium, Microbacterium, Pseudomonas, Agrobacterium, Alicyclobacillus, Anabaena, Anacystis, Arthrobacter, Azotobacter, Chromatium, Erwinia, Methylobacterium, Phormidium, Rhodobacter, Rhodopseudomonas, Rhodospirillum, Scenedesmus, Streptomyces, Synechoccus and Zymomonas. Specific examples are Escherichia coli XL1-Blue, Escherichia coli XL2-Blue, Escherichia coli DH1, Escherichia coli DH5α, Escherichia coli MC1000, Escherichia coli KY3276, Escherichia coli W1485, Escherichia coli JM109, Escherichia coli HB101, Escherichia coli No. 49, Escherichia coli W3110, Escherichia coli NY49, Escherichia coli MP347, Escherichia coli NM522, Bacillus subtilis ATCC 33712, Bacillus megaterium, Bacillus sp. FERM BP-6030, Bacillus amyloliquefaciens, Bacillus coagulans, Bacillus licheniformis, Bacillus pumilus, Brevibacterium ammoniagenes, Brevibacterium immariophilum ATCC 14068, Brevibacterium saccharolyticum ATCC 14066, Brevibacterium flavum ATCC 14067, Brevibacterium lactofermentum ATCC 13869, Corynebacterium glutamicum ATCC 13032, Corynebacterium glutamicum ATCC 14297, Corynebacterium acetoacidophilum ATCC 13870, Microbacterium ammoniaphilum ATCC 15354, Serratia ficaria, Serratia fonticola, Serratia liquefaciens, Serratia marcescens, Pseudomonas sp. D-0110, Agrobacterium radiobacter, Agrobacterium rhizogenes, Agrobacterium rubi, Anabaena cylindrica, Anabaena doliolum, Anabaena flosaquae, Arthrobacter aurescens, Arthrobacter citreus, Arthrobacter globiformis, Arthrobacter hydrocarboglutamicus, Arthrobacter mysorens, Arthrobacter nicotianae, Arthrobacter paraffineus, Arthrobacter protophormiae, Arthrobacter roseoparaffinus, Arthrobacter sulfureus, Arthrobacter ureafaciens, Chromatium buderi, Chromatium tepidum, Chromatium vinosum, Chromatium warmingii, Chromatium fluviatile, Erwinia uredovora, Erwinia carotovora, Erwinia ananas, Erwinia herbicola, Erwinia punctata, Erwinia terreus, Methylobacterium rhodesianum, Methylobacterium extorquens, Phormidium sp.. ATCC 29409, Rhodobacter capsulatus, Rhodobacter sphaeroides, Rhodopseudomonas blastica, Rhodopseudomonas marina, Rhodopseudomonas palustris, Rhodospirillum rubrum, Rhodospirillum salexigens, Rhodospirillum salinarum, Streptomyces ambofaciens, Streptomyces aureofaciens, Streptomyces aureus, Streptomyces fungicidicus, Streptomyces griseochromogenes, Streptomyces griseus, Streptomyces lividans, Streptomyces olivogriseus, Streptomyces rameus, Streptomyces tanashiensis, Streptomyces vinaceus and Zymomonas mobilis. Preferred procaryotes include microorganisms belonging to the genera Escherichia, Bacillus, Streptomyces and Corynebacterium.

Introduction of the recombinant DNA can be carried out by any of the methods for introducing DNA into the above host cells, for example, the method using calcium ion [Proc. Natl. Acad. Sci. USA, 69, 2110 (1972)], the protoplast method (Japanese Published Unexamined Patent Application No. 248394/88) and electroporation [Nucleic Acids Res., 16, 6127 (1988)].

When a yeast strain is used as the host cell, YEp13 (ATCC 37115), YEp24 (ATCC 37051), YCp50 (ATCC 37419), pHS19, pHS15, etc. can be used as the expression vector.

As the promoter, any promoters capable of functioning in yeast strains can be used. Suitable promoters include PH05 promoter, PGK promoter, GAP promoter, ADH promoter, gal 1 promoter, gal 10 promoter, heat shock polypeptide promoter, MFα1 promoter and CUP 1 promoter.

Examples of suitable host cells are yeast strains belonging to the genera Saccharomyces, Schizosaccharomyces, Kluyveromyces, Trichosporon, Schwanniomyces, Pichia and Candida, specifically, Saccharomyces cerevisiae, Schizosaccharomyces pombe, Kluyveromyces lactis, Trichosporon pullulans, Schwanniomyces alluvius, Pichia pastoris and Candida utilis.

Introduction of the recombinant DNA can be carried out by any of the methods for introducing DNA into yeast, for example, electroporation [Methods Enzymol., 194, 182 (1990)], the spheroplast method [Proc. Natl. Acad. Sci. USA, 81, 4889 (1984)] and the lithium acetate method [J. Bacteriol., 153, 163 (1983)].

When an animal cell is used as the host cell, pcDNAI, pcDM8 (commercially available from Funakoshi Co., Ltd.), pAGE107 (Japanese Published Unexamined Patent Application No. 22979/91), pAS3-3 (Japanese Published Unexamined Patent Application No. 227075/90), pCDM8 [Nature, 329, 840 (1987)], pcDNAI/Amp (Invitrogen Corp.), pREP4 (Invitrogen Corp.), pAGE103 [J. Biochem., 101, 1307 (1987)], pAGE210, pAMo, pAMoA, etc. can be used as the expression vector.

As the promoter, any promoters capable of functioning in animal cells can be used. Suitable promoters include the promoter of IE (immediate early) gene of cytomegalovirus (CMV), SV40 early promoter, metallothionein promoter, the promoter of a retrovirus, heat shock promoter, SRα promoter, etc. The enhancer of IE gene of human CMV may be used in combination with the promoter.

Examples of suitable host cells are mouse myeloma cells, rat myeloma cells, mouse hybridomas, human-derived Namalwa cells and Namalwa KJM-1 cells, human embryonic kidney cells, human leukemia cells, African green monkey kidney cells, Chinese hamster-derived CHO cells, and HBT5637 (Japanese Published Unexamined Patent Application No. 299/88).

The mouse myeloma cells include SP2/0 and NSO; the rat myeloma cells include YB2/0; the human embryonic kidney cells include HEK293 (ATCC CRL-1573); the human leukemia cells include BALL-1; and the African green monkey kidney cells include COS-1 and COS-7.

Introduction of the recombinant DNA can be carried out by any of the methods for introducing DNA into animal cells, for example, electroporation [Cytotechnology, 3, 133 (1990)], the calcium phosphate method (Japanese Published Unexamined Patent Application No. 227075/90), lipofection [Proc. Natl. Acad. Sci. USA, 84, 7413 (1987)], and the method described in Virology, 52, 456 (1973). ,

When an insect cell is used as the host cell, the protein can be produced by using the methods described in Baculovirus Expression Vectors, A Laboratory Manual, W. H. Freeman and Company, New York (1992); Current Protocols in Molecular Biology; Molecular Biology, A Laboratory Manual; Bio/Technology, 6, 47 (1988), etc.

That is, the recombinant gene transfer vector and a baculovirus are cotransfected into insect cells to obtain a recombinant virus in the culture supernatant of the insect cells, and then insect cells are infected with the recombinant virus, whereby the protein can be produced.

The gene transfer vectors useful in this method include pVL1392, pVL1393 and pBlueBacIII (products of Invitrogen Corp.).

An example of the baculovirus is Autographa californica nuclear polyhedrosis virus, which is a virus infecting insects belonging to the family Barathra.

Examples of the insect cells are ovarian cells of Spodoptera frugiperda, ovarian cells of Trichoplusia ni, and cultured cells derived from silkworm ovary.

The ovarian cells of Spodoptera frugiperda include Sf9 and Sf21 (Baculovirus Expression Vectors, A Laboratory Manual); the ovarian cells of Trichoplusia ni include High 5 and BTI-TN-5B1-4 (Invitrogen Corp.); and the cultured cells derived from silkworm ovary include Bombyx mori N4.

Cotransfection of the above recombinant gene transfer vector and the above baculovirus into insect cells for the preparation of the recombinant virus can be carried out by the calcium phosphate method (Japanese Published Unexamined Patent Application No. 227075/90), lipofection [Proc. Natl. Acad. Sci. USA, 84, 7413 (1987)], etc.

When a plant cell is used as the host cell, Ti plasmid, tobacco mosaic virus vector, etc. can be used as the expression vector.

As the promoter, any promoters capable of functioning in plant cells can be used. Suitable promoters include 35S promoter of cauliflower mosaic virus (CaMV), rice actin 1 promoter, etc.

Examples of suitable host cells are cells of plants such as tobacco, potato, tomato, carrot, soybean, rape, alfalfa, rice, wheat and barley.

Introduction of the recombinant vector can be carried out by any of the methods for introducing DNA into plant cells, for example, the method using Agrobacterium (Japanese Published Unexamined Patent Application Nos. 140885/84 and 70080/85, WO94/00977), electroporation (Japanese Published Unexamined Patent Application No. 251887/85) and the method using particle gun (gene gun) (Japanese Patent Nos. 2606856 and 2517813).

When the DNA is expressed in yeast, an animal cell, an insect cell or a plant cell, a glycosylated protein can be obtained.

The protein of the present invention can be produced by culturing the transformant obtained as above in a medium, allowing the protein of the present invention to form and accumulate in the culture, and recovering the protein from the culture.

The host of the above transformant for producing the protein of the present invention may be any bacterium, yeast, animal cell, insect cell, plant cell or the like, but is preferably a bacterium, more preferably a microorganism belonging to the genus Escherichia, and further preferably a microorganism belonging to Escherichia coli.

Culturing of the above transformant in a medium can be carried out by conventional methods for culturing the host.

For the culturing of the transformant obtained by using a procaryote such as Escherichia coli or a eucaryote such as yeast as the host, any of natural media and synthetic media can be used insofar as it is a medium suitable for efficient culturing of the transformant which contains carbon sources, nitrogen sources, inorganic salts, etc. which can be assimilated by the host used.

As the carbon sources, any carbon sources that can be assimilated by the host can be used. Examples of suitable carbon sources include carbohydrates such as glucose, fructose, sucrose, molasses containing them, starch and starch hydrolyzate; organic acids such as acetic acid and propionic acid; and alcohols such as ethanol and propanol.

As the nitrogen sources, ammonia, ammonium salts of organic or inorganic acids such as ammonium chloride, ammonium sulfate, ammonium acetate and ammonium phosphate, and other nitrogen-containing compounds can be used as well as peptone, meat extract, yeast extract, corn steep liquor, casein hydrolyzate, soybean cake, soybean cake hydrolyzate, and various fermented microbial cells and digested products thereof.

Examples of the inorganic salts include potassium dihydrogenphosphate, dipotassium hydrogenphosphate, magnesium phosphate, magnesium sulfate, sodium chloride, ferrous sulfate, manganese sulfate, copper sulfate and calcium carbonate.

Culturing is usually carried out under aerobic conditions, for example, by shaking culture or submerged spinner culture under aeration. The culturing temperature is preferably 15 to 40°C, and the culturing period is usually 5 hours to 7 days. The pH is maintained at 3.0 to 9.0 during the culturing. The pH adjustment is carried out by using an organic or inorganic acid, an alkali solution, urea, calcium carbonate, ammonia, etc.

If necessary, antibiotics such as ampicillin and tetracycline may be added to the medium during the culturing.

When a microorganism transformed with an expression vector comprising an inducible promoter is cultured, an inducer may be added to the medium, if necessary. For example, in the case of a microorganism transformed with an expression vector comprising lac promoter, isopropyl-β-D-thiogalactopyranoside or the like may be added to the medium; and in the case of a microorganism transformed with an expression vector comprising trp promoter, indoleacrylic acid or the like may be added.

For the culturing of the transformant obtained by using an animal cell as the host cell, generally employed media such as RPMI1640 medium [J. Am. Med. Assoc., 199, 519 (1967)], Eagle's MEM [Science, 122, 501 (1952)], DMEM [Virology, 8, 396 (1959)] and 199 medium [Proc. Soc. Biol. Med., 73, 1 (1950)], media prepared by adding fetal calf serum or the like to these media, etc. can be used as the medium.

Culturing is usually carried out at pH 6 to 8 at 25 to 40°C for 1 to 7 days in the presence of 5% CO₂.

If necessary, antibiotics such as kanamycin, penicillin and streptomycin may be added to the medium during the culturing.

For the culturing of the transformant obtained by using an insect cell as the host cell, generally employed media such as TNM-FH medium (PharMingen, Inc.), Sf-900 II SFM medium (Life Technologies, Inc.), ExCell 400 and ExCell 405 (JRH Biosciences, Inc.) and Grace's Insect Medium [Nature, 195, 788 (1962)] can be used as the medium.

Culturing is usually carried out at pH 6 to 7 at 25 to 30°C for 1 to 5 days.

If necessary, antibiotics such as gentamicin may be added to the medium during the culturing.

The transformant obtained by using a plant cell as the host cell may be cultured in the form of cells as such or after differentiation into plant cells or plant organs. For the culturing of such transformant, generally employed media such as Murashige-Skoog (MS) medium and White medium, media prepared by adding phytohormones such as auxin and cytokinin to these media, etc. can be used as the medium.

Culturing is usually carried out at pH 5 to 9 at 20 to 40° C for 3 to 60 days.

If necessary, antibiotics such as kanamycin and hygromycin may be added to the medium during the culturing.

As described above, the protein of the present invention can be produced by culturing, according to a conventional culturing method, the transformant derived from a microorganism, an insect cell, an animal cell or a plant cell and carrying a recombinant DNA prepared by ligating the DNA of the present invention or the DNA used in the production process of the present invention to an expression vector, allowing the protein to form and accumulate, and recovering the protein from the culture.

The protein of the present invention may be produced by intracellular production by host cells, extracellular secretion by host cells or production on outer membranes by host cells. The kind of the host cells used may be selected and the structure of the protein to be produced may be altered according to the production method.

When the protein of the present invention is produced in host cells or on outer membranes of host cells, it is possible to force the protein to be secreted outside the host cells by applying the method of Paulson, et al. [J. Biol. Chem., 264, 17619 (1989)], the method of Lowe, et al. [Proc. Natl. Acad. Sci. USA, 86, 8227 (1989); Genes Develop., 4, 1288 (1990)], or the methods described in Japanese Published Unexamined Patent Application No. 336963/93, WO94/23021, etc.

That is, extracellular secretion of the protein of the present invention by host cells can be caused by producing it in the form of a protein in which a signal peptide is added upstream of a protein containing the active site of the protein of the present invention by the use of recombinant DNA techniques.

It is also possible to increase the protein production by utilizing a gene amplification system using a dihydrofolate reductase gene or the like according to the method described in Japanese Published Unexamined Patent Application No. 227075/90.

Further, the protein of the present invention can be produced using an animal having an introduced gene (non-human transgenic animal) or a plant having an introduced gene (transgenic plant) constructed by redifferentiation of animal or plant cells carrying the introduced gene.

When the transformant producing the protein of the present invention is an animal or plant, the protein can be produced by raising or culturing the animal or plant in a usual manner, allowing the protein to form and accumulate therein, and recovering the protein from the animal or plant.

Production of the protein of the present invention using an animal can be carried out, for example, by producing the protein in an animal constructed by introducing the gene according to known methods [Am. J. Clin. Nutr., 63, 639S (1996); Am. J. Clin. Nutr., 63, 627S (1996); Bio/Technology, 9, 830 (1991)].

In the case of an animal, the protein of the present invention can be produced, for example, by raising a non-human transgenic animal carrying the introduced DNA of the present invention or DNA used in the production process of the present invention, allowing the protein to form and accumulate in the animal, and recovering the protein from the animal. The places where the protein is formed and accumulated include milk (Japanese Published Unexamined Patent Application No. 309192/88), egg, etc. of the animal. As the promoter in this process, any promoters capable of functioning in an animal can be used. Preferred promoters include mammary gland cell-specific promoters such as α casein promoter, β casein promoter, β lactoglobulin promoter and whey acidic protein promoter.

Production of the protein of the present invention using a plant can be carried out, for example, by culturing a transgenic plant carrying the introduced DNA encoding the protein of the present invention according to known methods [Soshiki Baiyo (Tissue Culture), 20, (1994); Soshiki Baiyo, 21, (1995); Trends Biotechnol., 15, 45 (1997)], allowing the protein to form and accumulate in the plant, and recovering the protein from the plant.

The protein of the present invention produced by using the transformant producing the protein of the present invention can be isolated and purified by conventional methods for isolating and purifying enzymes.

For example, when the protein of the present invention is produced in a soluble form in cells, the cells are recovered by centrifugation after the completion of culturing and suspended in an aqueous buffer, followed by disruption using a sonicator, French press, Manton Gaulin homogenizer, Dynomill or the like to obtain a cell-free extract.

A purified protein preparation can be obtained by centrifuging the cell-free extract to obtain the supernatant and then subjecting the supernatant to ordinary means for isolating and purifying enzymes, e.g., extraction with a solvent, salting-out with ammonium sulfate, etc., desalting, precipitation with an organic solvent, anion exchange chromatography using resins such as diethylaminoethyl (DEAE)-Sepharose and DIAION HPA-75 (Mitsubishi Chemical Corporation), cation exchange-chromatography using resins such as S-Sepharose FF (Pharmacia), hydrophobic chromatography using resins such as butyl Sepharose and phenyl Sepharose, gel filtration using a molecular sieve, affinity chromatography, chromatofocusing, and electrophoresis such as isoelectric focusing, alone or in combination.

When the protein is produced as an inclusion body in cells, the cells are similarly recovered and disrupted, followed by centrifugation to obtain a precipitate fraction. After the protein is recovered from the precipitate fraction by an ordinary method, the inclusion body of the protein is solubilized with a protein-denaturing agent.

The solubilized protein solution is diluted with or dialyzed against a solution containing no protein-denaturing agent or a solution containing the protein-denaturing agent at such a low concentration that denaturation of protein is not caused, whereby the protein is renatured to have normal conformation. Then, a purified protein preparation can be obtained by the same isolation and purification steps as described above.

When the protein of the present invention or its derivative such as a glycosylated form is extracellularly secreted, the protein or its derivative such as a glycosylated form can be recovered in the culture supernatant.

That is, the culture is treated by the same means as above, e.g., centrifugation, to obtain a soluble fraction. A purified protein preparation can be obtained from the soluble fraction by using the same isolation and purification methods as described above.

Examples of the proteins obtained in the above manner are proteins consisting of the amino acid sequences shown in SEQ ID_NOS: 1 and 2.

It is also possible to produce the protein of the present invention as a fusion protein with another protein and to purify it by affinity chromatography using a substance having affinity for the protein to be fused.

Examples of the proteins to be fused include β-galactosidase, protein A, IgG-binding region of protein A, chloramphenicol acetyltransferase, poly(Arg), poly(Glu), protein G, maltose-binding protein, glutathione S-transferase, polyhistidine chain (His-tag), S peptide, DNA-binding protein domain, Tac antigen, thioredoxin, green fluorescent protein, FLAG peptide and arbitrary antibody epitopes [Akio Yamakawa, Experimental Medicine, 13, 469-474 (1995)].

Examples of the substances having affinity for the above proteins to be fused include antibodies recognizing β-galactosidase, protein A, immunoglobulin G-binding region of protein A, chloramphenicol acetyltransferase, poly(Arg), poly(Glu), protein G, maltose-binding protein, glutathione S-transferase, polyhistidine chain (His-tag), S peptide, DNA-binding protein domain, Tac antigen, thioredoxin, green fluorescent protein, FLAG peptide and arbitrary antibody epitopes, such as immunoglobulin G.

The protein of the present invention can also be produced by chemical synthetic methods such as the Fmoc method (the fluorenylmethyloxycarbonyl method) and the tBoc method (the t-butyloxycarbonyl method) based on the amino acid information on the protein obtained above. Further, the protein can be chemically synthesized by using peptide synthesizers from Advanced ChemTech, Perkin-Elmer, Pharmacia, Protein Technology Instrument, Synthecell-Vega, PerSeptive, Shimadzu Corporation, etc.

### (iii) Process for Producing a Dipeptide of the Present Invention

### (1) Enzymatic Process

An example of the enzymatic process for producing a dipeptide is a process which comprises: allowing the protein or the protein for dipeptide synthesis of the present invention, one or more kinds of amino acids, and ATP to be present in an aqueous medium; allowing a dipeptide represented by formula (I) to form and accumulate in the medium; and recovering the dipeptide from the medium.

One or more kinds, preferably one or two kinds of amino acids used as substrates in the above process are amino acids selected from the group consisting of L- or D-form of amino acids, glycine (Gly), β-alanine (βAla) and derivatives thereof, preferably amino acids selected from the group consisting of L-amino acids, Gly, βAla and derivatives thereof, which can be used in any combination. Examples of L- or D-form of amino acids are alanine (Ala), glutamine (Gln), glutamic acid (Glu), valine (Val), leucine (Leu), isoleucine (Ile), proline (Pro), phenylalanine (Phe), tryptophan (Trp), methionine (Met), serine (Ser), threonine (Thr), cysteine (Cys), asparagine (Asn), tyrosine (Tyr), lysine (Lys), arginine (Arg), histidine (His), aspartic acid (Asp), α-aminobutyric acid (α-AB), azaserine, theanine, 4-hydroxyproline (4-HYP), 3-hydroxyproline (3-HYP), ornithine (Orn), citrulline (Cit) and 6-diazo-5-oxo-norleucine. Examples of L-form of amino acids are L-Ala, L-Gln, L-Glu, Gly, L-Val, L-Leu, L-Ile, L-Pro, L-Phe, L-Trp, L-Met, L-Ser, L-Thr, L-Cys, L-Asn, L-Tyr, L-Lys, L-Arg, L-His, L-Asp, L-α-AB, L-azaserine, L-theanine, L-4-HYP, L-3-HYP, L-Orn, L-Cit and L-6-diazo-5-oxo-norleucine.

Examples of the derivatives of amino acids include hydroxyamino acids (e.g., β-hydroxyglutamine, β-hydroxyglutamic acid, γ-hydroxyglutamic acid, α-hydroxyglycine, β-hydroxyvaline, γ-hydroxyvaline, β-hydroxyleucine, γ-hydroxyleucine, δ-hydroxyleucine, β-hydroxyisoleucine, γ-hydroxyisoleucine, 3-hydroxyproline, 4-hydroxyproline, β-hydroxyphenylalanine, 3,4-dihydroxyphenylalanine, 2,4,5-trihydroxyphenylalanine, β-hydroxytryptophan, 5-hydroxytryptophan, α-hydroxymethionine, β-hydroxyserine, γ-hydroxythreonine, S-hydroxycysteine, β-hydroxyasparagine, β-hydroxytyrosine, β-hydroxylysine, γ-hydroxylysine, δ-hydroxylysine, N-hydroxylysine, β-hydroxyarginine, δ-hydroxyarginine, N-hydroxyarginine, β-hydroxyhistidine, β-hydroxyaspartic acid, β-hydroxyornithine, γ-hydroxyornithine and N-hydroxyornithine) and N-methyl amino acids (e.g., N-methyl-alanine, N-methyl-glutamine, N-methyl-glutamic acid, N-methyl-glycine, N-methyl-valine, N-methyl-leucine, N-methyl-isoleucine, N-methyl-proline, N-methylphenylalanine, N-methyl-tryptophan, N-methyl-methionine, N-methyl-serine, N-methyl-threonine, N-methyl-cysteine, N-methyl-asparagine, N-methyl-tyrosine, N-methyl-lysine, N-methyl-arginine, N-methyl-histidine, N-methyl-aspartic acid and N-methyl-ornithine).

More preferred one or two kinds of amino acids used in the above process are one or two kinds of amino acids selected from the group consisting of L-Ala, L-Leu and L-Phe, and further preferred are one kind of amino acid selected from the group consisting of L-Ala, L-Leu and L-Phe, and a combination of two kinds of amino acids L-Leu and L-Phe.

In the above process, the protein or the protein for dipeptide synthesis of the present invention is added in an amount of 0.01 to 100 mg, preferably 0.1 mg to 10 mg per mg of amino acid used as a substrate.

In the above process, the amino acid used as a substrate is added to the aqueous medium at the start or in the course of reaction to give a concentration of 0.1 to 500 g/l, preferably 0.2 to 200 g/l in the total amount.

In the above process, ATP used as an energy source is used at a concentration of 0.5 mmol/l to 10 mol/l.

The aqueous medium used in the above process may comprise any components and may have any composition so far as the dipeptide-forming reaction is not inhibited. Suitable aqueous media include water and buffers such as phosphate buffer, carbonate buffer, acetate buffer, borate buffer, citrate buffer and Tris buffer. The aqueous medium may comprise alcohols such as methanol and ethanol, esters such as ethyl acetate, ketones such as acetone, and amides such as acetamide.

The dipeptide-forming reaction is carried out in the aqueous medium at pH 5 to 11, preferably pH 6 to 10, at 20 to 50°C, preferably 25 to 45°C, for 2 to 150 hours, preferably 6 to 120 hours.

Examples of the dipeptides produced by the above process are the dipeptides represented by formula (I). Preferred examples are dipeptides represented by formula (I) wherein R¹ and R², which may be the same or different, each represent L- or D-form of amino acid, glycine, β-alanine or a derivative thereof, and more preferred are those wherein R¹ and R² each represent L-form of amino acid, glycine, β-alanine or a derivative thereof.

Examples of L-form of amino acids and derivatives thereof include the substances mentioned above.

Examples of further preferred dipeptides produced by the above process are L-leucyl-L-phenylalanine, L-phenylalanyl-L-leucine, L-alanyl-L-alanine, L-leucyl-L-leucine and L-phenylalanyl-L-phenylalanine.

### (2) Process Using a Culture of a Transformant or a Microorganism or a Treated Matter of the Culture as an Enzyme Source

An example of the process for producing a dipeptide using a culture of a transformant or a microorganism or a treated matter of the culture as an enzyme source is a process which comprises: allowing an enzyme source and one or more kinds of amino acids to be present in an aqueous medium, said enzyme source being a culture of a transformant having the ability to produce the protein or the protein for dipeptide synthesis of the present invention or a microorganism having the ability to produce the protein or the protein for dipeptide synthesis of the present invention, or a treated matter of the culture; allowing a dipeptide represented by formula (I) to form and accumulate in the medium; and recovering the dipeptide from the medium.

The transformants useful in the above process include the transformants producing the protein or the protein for dipeptide synthesis of the present invention that can be produced by the method of the above (ii). As the hosts of the transformants, bacteria, yeast, animal cells, insect cells, plant cells, etc. can be used. Preferred hosts are bacteria, among which microorganisms belonging to the genera Escherichia, Bacillus, Streptomyces and Corynebacterium are more preferred.

Preferred microorganisms belonging to the genus Escherichia include those belonging to Escherichia coli; preferred microorganisms belonging to the genus Bacillus include those belonging to Bacillus subtilis and Bacillus megaterium; preferred microorganisms belonging to the genus Streptomyces include those belonging to Streptomyces lividans; and preferred microorganisms belonging to the genus Corynebacterium include those belonging to Corynebacterium glutamicum and Corynebacterium ammoniagenes.

The microorganism used in the above process may be any microorganism having the ability to produce the protein or the protein for dipeptide synthesis of the present invention, but is preferably a microorganism belonging to the genus Streptomyces, more preferably a microorganism belonging to the genus Streptomyces which has albonoursin-synthesizing activity, further preferably a microorganism belonging to a species selected from the group consisting of Streptomyces albulus and Streptomyces noursei, most preferably Streptomyces albulus IFO14147, Streptomyces noursei ATCC 11455 or Streptomyces noursei IFO15452.

As the treated matter of a culture, any treated matters that underwent any known treatment may be used insofar as they have the same activity as the culture. Examples of the treated matters of the culture include concentrated culture, dried culture, cells obtained by centrifuging the culture, dried cells, freeze-dried cells, surfactant-treated cells, solvent-treated cells, enzyme-treated cells and immobilized cells which contain living cells, and ultrasonic-treated cells, mechanically disrupted cells, protein fractionation of the cells and enzyme preparations obtained from the cells by extraction.

In the above process, the kinds of amino acids used as substrates, their concentrations, the time of their addition, and the dipeptides produced are similar to those in the enzymatic process described in the above (iii) (1).

In the process using a culture of a microorganism or a treated matter of the culture as an enzyme source, the culture broth of the transformant or microorganism used as an enzyme source can also be used as the aqueous medium in addition to the aqueous media used in the enzymatic process described in the above (iii) (1).

Further, in the above process, ATP or compounds which can be metabolized by the transformant or microorganism to produce ATP, for example, sugars such as glucose, alcohols such as ethanol, and organic acids such as acetic acid may be added, as ATP source, to the aqueous medium according to need.

If necessary, a surfactant or an organic solvent may further be added to the aqueous medium. Any surfactant that promotes the formation of a dipeptide can be used. Suitable surfactants include nonionic surfactants such as polyoxyethylene octadecylamine (e.g., Nymeen S-215, NOF Corporation), cationic surfactants such as cetyltrimethylammonium bromide and alkyldimethylbenzylammonium chloride (e.g., Cation F2-40E, NOF Corporation), anionic surfactants such as lauroyl sarcosinate, and tertiary amines such as alkyldimethylamine (e.g., Tertiary Amine FB, NOF Corporation), which may be used alone or in combination. The surfactant is usually used at a concentration of 0.1 to 50 g/l. As the organic solvent, xylene, toluene, aliphatic alcohols, acetone, ethyl acetate, etc. may be used usually at a concentration of 0.1 to 50 ml/l.

When the culture or a treated matter of the culture is used as the enzyme source, the amount of the enzyme source to be added varies according to its specific activity, etc., but is, for example, 5 to 1000 mg (wet cell weight), preferably 10 to 400 mg per mg of amino acid used as a substrate.

The dipeptide-forming reaction is carried out in the aqueous medium at pH 5 to 11, preferably pH 6 to 10, at 20 to 65°C, preferably 25 to 55°C, more preferably 30 to 45°C, for 1 minute to 150 hours, preferably 3 minutes to 120 hours, more preferably 30 minutes to 100 hours.

In the processes described in the above (iii) (1) and (2), recovery of the dipeptide formed and accumulated in the aqueous medium can be carried out by ordinary methods using active carbon, ion-exchange resins, etc. or by means such as extraction with an organic solvent, crystallization, thin layer chromatography and high performance liquid chromatography.

Certain embodiments of the present invention are illustrated in the following examples. These examples are not to be construed as limiting the scope of the invention.

### Example 1

### Acquisition of the albC Gene and Its Analogous Gene

The albC gene and its analogous gene were obtained from Streptomyces noursei and Streptomyces albulus based on the nucleotide sequence of the albC gene of Streptomyces noursei [Chemistry & Biol., 9, 1355 (2002)] in the following manner.

Streptomyces noursei IF015452 and Streptomyces albulus IF014147 were inoculated into KM73 medium [2 g/l yeast extract (Difco) and 10 g/l soluble starch (Wako Pure Chemical Industries, Ltd.)] containing 1% glycine and KP medium [15 g/l glucose, 10 g/l glycerol, 10 g/l polypeptone (Nihon Pharmaceutical Co., Ltd.), 10 g/l meat extract (Kyokuto Pharmaceutical Industrial Co., Ltd.) and 4 g/l calcium carbonate], respectively, and subjected to shaking culture overnight at 28°C. Streptomyces noursei IFO15452 and Streptomyces albulus IFO1417 were distributed by National Institute of Technology and Evaluation (NITE) Biological Resource Center (BRC) (2-5-8, Kazusakaniatari, Kisarazu-shi, Chiba 292-0818 Japan).

After the culturing, the chromosomal DNAs of the respective microorganisms were isolated and purified according to the method described in Genetic Manipulation of Streptomyces: a Laboratory Manual: John Innes Foundation.

On the basis of the nucleotide sequence of the albC gene, DNAs having the nucleotide sequences shown in SEQ ID NOS: 5 and 6 (hereinafter referred to as primer A and primer B, respectively) were synthesized by using a DNA synthesizer (Model 8905, PerSeptive Biosystems, Inc.). Primer A has a nucleotide sequence wherein a sequence containing the NcoI recognition sequence is added to the 5' end of a region containing the initiation codon of the albC gene on the chromosomal DNA of Streptomyces noursei. Primer B has a nucleotide sequence wherein a sequence containing the BglII recognition sequence is added to the 5' end of a nucleotide sequence complementary to a sequence containing the termination codon of the albC gene.

PCR was carried out using each of the chromosomal DNAs of Streptomyces noursei and Streptomyces albulus as a template and the above primer A and primer B as a set of primers. PCR was carried out for 30 cycles of 94°C for one minute, 55°C for 30 seconds and 72°C for one minute, using 50 µl of a reaction mixture comprising 0.1 µg of the chromosomal DNA as a template, 0.5 µmol/l each of the primers, 2.5 units of Ex Taq DNA polymerase (Takara Bio Inc.), 5 µl of buffer for Ex Taq DNA polymerase (10 x) (Takara Bio Inc.), 200 µmol/l each of dNTPs (dATP, dGTP, dCTP and dTTP) and 5 µl of dimethyl sulfoxide.

One-tenth of each of the resulting reaction mixtures was subjected to agarose gel electrophoresis to confirm that a ca. 0.7 kb DNA fragment was amplified. Then, the remaining reaction mixture was mixed with an equal amount of phenol/chloroform (1 vol/l vol) saturated with TE [10 mmol/l Tris-HCl (pH 8.0), 1 mmol/l EDTA]. The resulting solution was centrifuged, and the obtained upper layer was mixed with a two-fold volume of cold ethanol and allowed to stand at -80°C for 30 minutes. The resulting solution was centrifuged to precipitate DNA, and the obtained DNA was dissolved in 20 µl of TE.

Each of the thus obtained solutions (5 µl) was subjected to reaction to cleave the amplified DNA with restriction enzymes NcoI and BglII. DNA fragments were separated by agarose gel electrophoresis, and a 700 bp DNA fragment was recovered using GENECLEAN II Kit (BIO 101), respectively.

Expression vector pQE60 containing phage T5 promoter (Qiagen, Inc.) (0.2 µg) was cleaved with restriction enzymes NcoI and BglII. DNA fragments were separated by agarose gel electrophoresis, and a 3.4 kb DNA fragment was recovered in the same manner as above.

Each of the actinomycetes-derived 0.7 kb DNA fragments and the pQE60-derived 3.4 kb DNA fragment obtained above were subjected to ligation reaction using a ligation kit (Takara Bio Inc.) at 16°C for 16 hours.

Escherichia coli NM522 (Stratagene) was transformed using each ligation reaction mixture according to the method using calcium ion [Proc. Natl. Acad. Sci. USA, 69, 2110 (1972)], spread on LB agar medium [10 g/l Bacto-tryptone (Difco), 5 g/l yeast extract (Difco), 5 g/l sodium chloride and 20 g/l agar] containing 50 µg/ml ampicillin, and cultured overnight at 30°C.

A plasmid was extracted from a colony of each transformant that grew on the medium according to a known method, and the structure of each plasmid was analyzed using restriction enzymes. As a result, it was confirmed that expression vector pAL-nou containing the DNA derived from Streptomyces noursei at a position downstream of the phage T5 promoter and expression vector pAL-alb containing the DNA derived from Streptomyces albulus were obtained (Fig. 1).

The nucleotide sequence of each actinomycete-derived DNA inserted into the respective plasmid was determined by using a nucleotide sequencer (373A DNA Sequencer), whereby it was confirmed that pAL-alb contained DNA encoding a protein having the amino acid sequence shown in SEQ ID NO: 1, i.e. DNA having the nucleotide sequence shown in SEQ ID NO: 3, and pAL-nou contained DNA encoding a protein having the amino acid sequence shown in SEQ ID NO: 2, i.e. DNA having the nucleotide sequence shown in SEQ ID NO: 4.

### Example 2

### Production of Dipeptides Using Cells as an Enzyme Source

Escherichia coli NM522 carrying pAL-nou or pAL-alb obtained in Example 1 (Escherichia coli NM522/pAL-nou or NM522/pAL-alb) and Escherichia coli NM522 without a plasmid were respectively inoculated into 10 ml of LB medium [10 g/l Bacto-tryptone (Difco), 5 g/l yeast extract (Difco) and 5 g/l sodium chloride] containing 50 µg/ml ampicillin in a test tube (no addition of ampicillin in the case of a strain carrying no plasmid, hereinafter the same shall apply), and cultured at 30°C for 17 hours. Each of the resulting cultures (0.5 ml) was inoculated into 50 ml of LB medium in a 250-ml Erlenmeyer flask and subjected to shaking culture at 30°C for one hour. Then, isopropyl-β-D-thiogalactopyranoside (IPTG) was added to give a final concentration of 1 mmol/l, followed by further culturing for 4 hours. The resulting culture was centrifuged to obtain wet cells.

A reaction mixture (3.0 ml) comprising 100 mg/ml (final concentration) wet cells, 60 mmol/l potassium phosphate buffer (pH 7.2), 10 mmol/l magnesium chloride, 10 mmol/l ATP, 1 g/l L-Leu and 1 g/l L-Phe was prepared, and reaction was carried out at 30°C. One hour after the start of the reaction, the reaction mixture was sampled and acetonitrile was added thereto to give a concentration of 20% (v/v). Then, the obtained reaction product was analyzed by HPLC under the following conditions.
Separation column: ODS-HA column (YMC Co., Ltd.)
Eluent: 30% (v/v) acetonitrile
Flow rate: 0.6 ml/min
Detection: ultraviolet absorption at 215 nm

As a result, it was confirmed that 36.7 mg/l cyclo(L-Leu-L-Phe) was accumulated in the reaction mixture of Escherichia coli NM522/pAL-nuo. However, no cyclo(L-Leu-L-Phe) was detected in the reaction mixture of Escherichia coli NM522. The same reaction mixtures were analyzed by HPLC under the following conditions to measure straight-chain dipeptides L-leucyl-L-phenylalanine (L-Leu-L-Phe) and L-phenylalanyl-L-leucine (L-Phe-L-Leu).

The straight-chain dipeptides were derivatized by the F-moc method and then analyzed by HPLC.
Separation column: ODS-HG-5 column (Nomura Kagaku Co., Ltd.)
Eluent:
   Solution A: 6 ml/l acetic acid and 20% (v/v) acetonitrile (pH adjusted to 4.8 with triethylamine)
   Solution B: 6 ml/l acetic acid and 70% (v/v) acetonitrile (pH adjusted to 4.8 with triethylamine)
Flow rate: 0.6 ml/min
Detection:
   Excitation wavelength: 254 nm
   Fluorescence wavelength: 630 nm
Eluent mixture ratio: shown in Table 1

**Table 1**

| Time passage (minute) | B% |
|---|---|
| 0 | 20 |
| 5 | 60 |
| 35 | 90 |
| 36 | 20 |
| 40 | 20 |

As a result, it was confirmed that 21.9 mg/l L-Leu-L-Phe and 12.0 mg/l L-Phe-L-Leu were accumulated in the reaction mixture of Escherichia coli NM522/pAL-nuo and no straight-chain dipeptide was detected in the reaction mixture of Escherichia coli NM522 used as a control strain. This result revealed that the cyclodipeptide-synthesizing enzyme obtained by the present invention has the ability to synthesize straight-chain dipeptides.

### Example 3

### Production of Dipeptides Using the Purified Enzyme (1)

Escherichia coli NM522/pAL-nuo was cultured in the same manner as in Example 2. After the completion of the culturing, centrifugation was carried out to obtain wet cells. The obtained wet cells were washed with a 60 mmol/l potassium phosphate buffer (pH 7.2) and suspended in a 20 mmol/l potassium phosphate buffer containing 10 mmol/l imidazole. The resulting suspension was subjected to ultrasonication at 4°C to obtain a disrupted cell suspension. The obtained suspension (10 ml: containing 0.863 mg of protein) was passed through a His-tag purification column (Amersham Biosciences K.K.) and then 15 ml of a 20 mmol/l potassium phosphate buffer containing 10 mmol/l imidazole was passed through the column for washing to purify a His-tagged albC protein in the column. Then, 2 ml of a reaction mixture having the same composition as that in Example 2 [composition: 60 mmol/l potassium phosphate buffer (pH 7.2), 10 mmol/l magnesium chloride, 10 mmol/l ATP, 1 g/l L-Leu, 1 g/l L-Phe] was put into the column containing the His-tagged albC protein, followed by incubation at 30°C, during which the substrates were held in the column. After 24 hours, the reaction mixture in the column was eluted with 3 ml of a reaction mixture having the same composition, and the cyclodipeptide and dipeptides in the reaction mixture were determined in the same manner as in Example 2.

As a result, it was confirmed that 6.8 mg/l cyclo(L-Leu-L-Phe), 28.7 mg/l L-Leu-L-Phe and 18.5 mg/l L-Phe-L-Leu were formed. No cyclodipeptide or dipeptide was detected in the reaction mixture without ATP incubated in the same manner.

### Example 4

### Production of Dipeptides Using the Purified Enzyme (2)

Enzymatic reaction was carried out in the same manner as in Example 3 except that the amino acids as substrates were replaced by another amino acid, and the obtained product was analyzed. As the reaction mixture, a mixture having the same composition as that of Example 2 except that the amino acids as the substrates were replaced by 1 g/l L-Ala, L-Leu or L-Phe was used.

As a result, it was revealed that 9.41 mg/l L-Ala-L-Ala, 7.85 mg/l L-Leu-L-Leu and 5.20 mg/l L-Phe-L-Phe were respectively formed in 24 hours after the start of the reaction.

### SEQUENCE LISTING FREE TEXT

SEQ ID NO: 5 - Description of Artificial Sequence:
Synthetic DNA

SEQ ID NO: 6 - Description of Artificial Sequence:
Synthetic DNA

<110> KYOWA HAKKO KOGYO CO., LTD.

<120> Process for producing dipeptides

<130> 1679

<160> 6

<170> PatentIn Ver. 2.1

<210> 1
<211> 239
<212> PRT
<213> Streptomyces noursei IFO15452

<400> 1
Met Leu Ala Gly Leu Val Pro Ala Pro Asp His Gly Met Arg Glu Glu
1               5                   10                  15

Ile Leu Gly Asp Arg Ser Arg Leu Ile Arg Gln Arg Gly Glu His Ala
            20                  25                  30

Leu Ile Gly Ile Ser Ala Gly Asn Ser Tyr Phe Ser Gln Lys Asn Thr
        35                  40                  45

Val Met Leu Leu Gln Trp Ala Gly Gln Arg Phe Glu Arg Thr Asp Val
    50                  55                  60

Val Tyr Val Asp Thr His Ile Asp Glu Met Leu Ile Ala Asp Gly Arg
65                  70                  75                  80

Ser Ala Gln Glu Ala Glu Arg Ser Val Lys Arg Thr Leu Lys Asp Leu
                85                  90                  95

Arg Arg Arg Leu Arg Arg Ser Leu Glu Ser Val Gly Asp His Ala Glu
            100                 105                 110

Arg Phe Arg Val Arg Ser Leu Ser Glu Leu Gln Glu Thr Pro Glu Tyr
        115                 120                 125

Arg Ala Val Arg Glu Arg Thr Asp Arg Ala Phe Glu Glu Asp Ala Glu
    130                 135                 140

Phe Ala Thr Ala Cys Glu Asp Met Val Arg Ala Val Val Met Asn Arg
145                 150                 155                 160

Pro Gly Asp Gly Val Gly Ile Ser Ala Glu His Leu Arg Ala Gly Leu
                165                 170                 175

Asn Tyr Val Leu Ala Glu Ala Pro Leu Phe Ala Asp Ser Pro Gly Val
            180                 185                 190

Phe Ser Val Pro Ser Ser Val Leu Cys Tyr His Ile Asp Thr Pro Ile
        195                 200                 205

Thr Ala Phe Leu Ser Arg Arg Glu Thr Gly Phe Arg Ala Ala Glu Gly
    210                 215                 220

Gln Ala Tyr Val Val Val Arg Pro Gln Glu Leu Ala Asp Ala Ala
225                 230                 235

<210> 2
<211> 239
<212> PRT
<213> Streptomyces alborus IFO15452

<400> 2
Met Leu Ala Gly Leu Val Pro Ala Leu Asp His Ser Met Arg Glu Glu
1               5                   10                  15

Ile Leu Gly Asn Arg Gly Arg Lys Ile Arg Gln Arg Gly Glu His Ala

            20                  25                  30

Leu Ile Gly Ile Ser Ala Gly Asn Ser Tyr Phe Ser Gln Lys Asn Val
        35                  40                  45

Thr Met Leu Leu Gln Trp Ala Gly Gln His Phe Glu Arg Thr Asp Val
    50                  55                  60

Val Tyr Val Asp Thr His Ile Asp Asp Met Leu Met Ala Asp Gly Arg
65                  70                  75                  80

Ser Ala Gln Glu Ala Glu Lys Ser Val Lys Arg Thr Leu Lys Asp Leu
                85                  90                  95

Arg Arg Arg Leu Arg Arg Ser Leu Glu Ser Val Gly Asp His Ser Glu
            100                 105                 110

Arg Phe Arg Val Arg Ser Leu Ser Glu Ile Gln Glu Thr Pro Glu Tyr
        115                 120                 125

Arg Ala Ala Arg Glu Ser Thr Asp Arg Ala Phe Arg Glu Asp Gly Glu
    130                 135                 140

Phe Ala Thr Val Cys Glu Glu Met Val Arg Ala Val Val Met Asn Arg
145                 150                 155                 160

Pro Gly Asp Gly Val Asp Ile Ser Glu Glu His Leu Arg Ala Gly Leu
                165                 170                 175

Asn Tyr Val Leu Ala Glu Ala Pro Leu Phe Ala Asp Ser Pro Gly Val
            180                 185                 190

Phe Ser Val Pro Ser Ser Val Leu Cys Tyr His Ile Pro Thr Pro Val
        195                 200                 205

Ser Thr Phe Leu Ala His Arg Glu Thr Gly Phe Gln Ala Ala Gln Gly

210                 215                 220

Gln Ala Tyr Val Val Val Arg Pro Gln Glu Leu Ala Asp Ala Ala
225                 230                 235

<210> 3
<211> 717
<212> DNA
<213> Streptomyces noursei IFO15452

<400> 3
atg ctt gca ggc tta gtt ccc gcg ccg gac cac gga atg cgg gaa gaa     48
Met Leu Ala Gly Leu Val Pro Ala Pro Asp His Gly Met Arg Glu Glu
  1               5                  10                  15

ata ctt ggc gac cgc agc cga ttg atc cgg caa cgc ggt gag cac gcc     96
Ile Leu Gly Asp Arg Ser Arg Leu Ile Arg Gln Arg Gly Glu His Ala
                 20                  25                  30

ctc atc gga atc agt gcg ggc aac agt tat ttc agc cag aag aac acc    144
Leu Ile Gly Ile Ser Ala Gly Asn Ser Tyr Phe Ser Gln Lys Asn Thr
             35                  40                  45

gtc atg ctg ctg caa tgg gcc ggg cag cgt ttc gag cgc acc gat gtc    192
Val Met Leu Leu Gln Trp Ala Gly Gln Arg Phe Glu Arg Thr Asp Val
         50                  55                  60

gtc tat gtc gac acc cac atc gac gag atg ctg atc gcc gac ggc cgc    240
Val Tyr Val Asp Thr His Ile Asp Glu Met Leu Ile Ala Asp Gly Arg
 65                  70                  75                  80

agc gcg cag gag gcc gag cgg tcg gtc aaa cgc acg ctc aag gat ctg    288
Ser Ala Gln Glu Ala Glu Arg Ser Val Lys Arg Thr Leu Lys Asp Leu
                 85                  90                  95

cgg cgc aga ctc cgg cgc tcg ctg gag agc gtg ggc gac cac gcc gag 336
Arg Arg Arg Leu Arg Arg Ser Leu Glu Ser Val Gly Asp His Ala Glu
            100                 105                 110

cgg ttc cgt gtc cgg tcc ctg tcc gag ctc cag gag acc cct gag tac 384
Arg Phe Arg Val Arg Ser Leu Ser Glu Leu Gln Glu Thr Pro Glu Tyr
        115                 120                 125

cgg gcc gta cgc gag cgc acc gac cgg gcc ttc gag gag gac gcc gaa 432
Arg Ala Val Arg Glu Arg Thr Asp Arg Ala Phe Glu Glu Asp Ala Glu
    130                 135                 140

ttc gcc acc gcc tgc gag gac atg gtg cgg gcc gtg gtg atg aac cgg 480
Phe Ala Thr Ala Cys Glu Asp Met Val Arg Ala Val Val Met Asn Arg
145                 150                 155                 160

ccc ggt gac ggc gtc ggc atc tcc gcg gaa cac ctg cgg gcc ggt ctg 528
Pro Gly Asp Gly Val Gly Ile Ser Ala Glu His Leu Arg Ala Gly Leu
                165                 170                 175

aac tac gtg ctg gcc gag gcc ccg ctc ttc gcg gac tcg ccc gga gtc 576
Asn Tyr Val Leu Ala Glu Ala Pro Leu Phe Ala Asp Ser Pro Gly Val
            180                 185                 190

ttc tcc gtc ccc tcc tcg gtg ctc tgc tac cac atc gac acc ccg atc 624
Phe Ser Val Pro Ser Ser Val Leu Cys Tyr His Ile Asp Thr Pro Ile
        195                 200                 205

acg gcg ttc ctg tcc cgg cgc gag acc ggt ttc cgg gcg gcc gag gga 672
Thr Ala Phe Leu Ser Arg Arg Glu Thr Gly Phe Arg Ala Ala Glu Gly
    210                 215                 220

cag gcg tac gtc gtc gtc agg ccc cag gag ctg gcc gac gcg gcc 717
Gln Ala Tyr Val Val Val Arg Pro Gln Glu Leu Ala Asp Ala Ala
225                 230                 235

<210> 4
<211> 717
<212> DNA
<213> Streptomyces alborus IFO15452

<400> 4
atg ctt gca ggc tta gtt ccc gcg ctg gac cac agc atg cgg gaa gaa 48
Met Leu Ala Gly Leu Val Pro Ala Leu Asp His Ser Met Arg Glu Glu
1               5                   10                  15

ata ctt ggc aat cgc ggc cga aag atc cgg caa cgc ggt gag cac gct 96
Ile Leu Gly Asn Arg Gly Arg Lys Ile Arg Gln Arg Gly Glu His Ala
            20                  25                  30

ctc att gga atc agt gcg ggc aac agt tat ttc agc cag aag aac gtc 144
Leu Ile Gly Ile Ser Ala Gly Asn Ser Tyr Phe Ser Gln Lys Asn Val
        35                  40                  45

acc atg ctg ctg caa tgg gcc ggg cag cat ttc gag cgc acg gat gtc 192
Thr Met Leu Leu Gln Trp Ala Gly Gln His Phe Glu Arg Thr Asp Val
    50                  55                  60

gtc tac gtg gac acg cac atc gac gac atg ctg atg gcg gac ggc cgc 240
Val Tyr Val Asp Thr His Ile Asp Asp Met Leu Met Ala Asp Gly Arg
65                  70                  75                  80

agc gcg cag gaa gcc gag aag tcg gtc aag cgc acg ctc aag gat ctg 288
Ser Ala Gln Glu Ala Glu Lys Ser Val Lys Arg Thr Leu Lys Asp Leu
                85                  90                  95

cgg cgc agg ctg cgg cgc tcg ttg gaa agc gtg ggc gac cac agc gag 336
Arg Arg Arg Leu Arg Arg Ser Leu Glu Ser Val Gly Asp His Ser Glu
            100                 105                 110

cgg ttc cgc gtc cgg tcc ctg tcc gag atc cag gag acc cct gag tac    384
Arg Phe Arg Val Arg Ser Leu Ser Glu Ile Gln Glu Thr Pro Glu Tyr
        115                 120                 125

cgg gcc gca cgc gag tcc acc gac cgg gcc ttc cgc gag gac ggc gag    432
Arg Ala Ala Arg Glu Ser Thr Asp Arg Ala Phe Arg Glu Asp Gly Glu
    130                 135                 140

ttc gcc acc gtc tgc gag gag atg gtg cgc gcc gtg gtg atg aac cgg    480
Phe Ala Thr Val Cys Glu Glu Met Val Arg Ala Val Val Met Asn Arg
145                 150                 155                 160

ccc ggt gac ggc gtc gac atc tcg gag gaa cac ctg cgg gcc ggt ctg    528
Pro Gly Asp Gly Val Asp Ile Ser Glu Glu His Leu Arg Ala Gly Leu
                165                 170                 175

aac tac gtg ctc gcc gag gcc ccg ctc ttc gcg gac tcg ccc ggc gtg    576
Asn Tyr Val Leu Ala Glu Ala Pro Leu Phe Ala Asp Ser Pro Gly Val
            180                 185                 190

ttc tcc gtc ccc tcg tcg gtg ctc tgc tac cac atc ccc acc ccg gta    624
Phe Ser Val Pro Ser Ser Val Leu Cys Tyr His Ile Pro Thr Pro Val
        195                 200                 205

tcg acg ttc ctg gcc cat cgc gag acc ggt ttc cag gcg gct cag ggt    672
Ser Thr Phe Leu Ala His Arg Glu Thr Gly Phe Gln Ala Ala Gln Gly
    210                 215                 220

cag gca tac gtc gtc gtc agg ccg cag gag ctg gcc gac gcg gcc        717
Gln Ala Tyr Val Val Val Arg Pro Gln Glu Leu Ala Asp Ala Ala
225                 230                 235

<210> 5
<211> 32
<212> DNA

<213> Artificial Sequence

<220>
<223> Description of Artificial Sequence: Synthetic DNA

<400> 5
agagccatgg gacttgcagg cttagttccc gc                                32

<210> 6
<211> 29
<212> DNA
<213> Artificial Sequence

<220>
<223> Description of Artificial Sequence: Synthetic DNA
agagagatct ggccgcgtcg gccagctcc                                    29

## Claims

1. A protein according to any of the following
[1] to [3], provided that a protein consisting of the amino acid sequence shown in SEQ ID NO: 1 is excluded:
[1] a protein having the amino acid sequence shown in SEQ ID NO: 2;
[2] a protein consisting of an amino acid sequence wherein one or more amino acid residues are deleted, substituted or added in the amino acid sequence shown in SEQ ID NO: 1 or 2 and having the activity to synthesize a dipeptide represented by formula (I):
R¹ - R² (I)
(wherein R¹ and R², which may be the same or different, each represent an amino acid); and
[3] a protein consisting of an amino acid sequence which has 65% or more homology to the amino acid sequence shown in SEQ ID NO: 1 or 2 and having the activity to synthesize a dipeptide represented by formula (I).

2. A protein for dipeptide synthesis according to any of the following [1] to [3]:
[1] a protein for dipeptide synthesis having the amino acid sequence shown in SEQ ID NO: 1;
[2] a protein for dipeptide synthesis consisting of an amino acid sequence wherein one or more amino acid residues are deleted, substituted or added in the amino acid sequence shown in SEQ ID NO: 1 and having the activity to synthesize a dipeptide represented by formula (I):
R¹ - R² (I)
(wherein R¹ and R², which may be the same or different, each represent an amino acid); and
[3] a protein for dipeptide synthesis consisting of an amino acid sequence which has 65% or more homology to the amino acid sequence shown in SEQ ID NO: 1 and having the activity to synthesize a dipeptide represented by formula (I).

3. A DNA according to any of the following [1] to [3], provided that a DNA consisting of the nucleotide sequence shown in SEQ ID NO: 3 is excluded:
[1] DNA encoding the protein according to Claim 1;
[2] DNA having the nucleotide sequence shown in SEQ ID NO: 4; and
[3] DNA which hybridizes with DNA having a nucleotide sequence complementary to the nucleotide sequence shown in SEQ ID NO: 4 under stringent conditions and which encodes a protein having the activity to synthesize a dipeptide represented by formula (I):
R¹ - R² (I)
(wherein R¹ and R², which may be the same or different, each represent an amino acid).

4. A recombinant DNA comprising the DNA according to Claim 3.

5. A transformant carrying the recombinant DNA according to Claim 4.

6. The transformant according to Claim 5, wherein the transformant is a transformant obtainable by using a microorganism as a host.

7. The transformant according to Claim 6, wherein the microorganism is a microorganism belonging to the genus Escherichia.

8. A process for producing the protein according to Claim 1, which comprises culturing the transformant according to any of Claims 5 to 7 in a medium, allowing the protein according to Claim 1 to form and accumulate in the culture, and recovering the protein from the culture.

9. A process for producing the protein according to Claim 1, which comprises culturing a microorganism having the ability to produce the protein according to Claim 1 in a medium, allowing the protein to form and accumulate in the culture, and recovering the protein from the culture.

10. The process according to Claim 9, wherein the microorganism is a microorganism belonging to the genus Streptomyces.

11. The process according to Claim 10, wherein the microorganism belonging to the genus Streptomyces is a microorganism belonging to the genus Streptomyces which has the ability to produce albonoursin.

12. The process according to Claim 11, wherein the microorganism belonging to the genus Streptomyces which has the ability to produce albonoursin is Streptomyces albulus or Streptomyces noursei.

13. A process for producing a dipeptide represented by formula (I):
R¹ - R² (I)
(wherein R¹ and R², which may be the same or different, each represent an amino acid), which comprises:
allowing the protein according to Claim 1 or the protein for dipeptide synthesis according to Claim 2, one or more kinds of amino acids, and ATP to be present in an aqueous medium;
allowing the dipeptide to form and accumulate in the medium; and
recovering the dipeptide from the medium.

14. A process for producing a straight-chain dipeptide represented by formula (I):
R¹- R² (I)
(wherein R¹ and R², which may be the same or different, each represent an amino acid), which comprises:
allowing an enzyme source and one or more kinds of amino acids to be present in an aqueous medium, said enzyme source being a culture or a treated matter of the culture selected from the group consisting of the following [1] to [3]:
[1] a culture of the transformant according to any of Claims 5 to 7 or a treated matter of the culture;
[2] a culture of a microorganism having the ability to produce the protein according to Claim 1 or a treated matter of the culture; and
[3] a culture of a microorganism having the ability to produce the protein for dipeptide synthesis according to Claim 2 or a treated matter of the culture;
allowing the dipeptide to form and accumulate in the medium; and
recovering the dipeptide from the medium.

15. The process according to Claim 14, wherein the microorganism having the ability to produce the protein according to Claim 1 is a microorganism belonging to the genus Streptomyces.

16. The process according to Claim 14, wherein the microorganism having the ability to produce the protein for dipeptide synthesis according to Claim 2 is a microorganism belonging to the genus Streptomyces.

17. The process according to Claim 15 or 16, wherein the microorganism belonging to the genus Streptomyces is a microorganism belonging to the genus Streptomyces which has the ability to produce albonoursin.

18. The process according to Claim 17, wherein the microorganism belonging to the genus Streptomyces which has the ability to produce albonoursin is a microorganism belonging to Streptomyces albulus or Streptomyces noursei.

19. The process according to Claim 14, wherein the microorganism having the ability to produce the protein for dipeptide synthesis according to Claim 2 is a microorganism transformed with DNA encoding the protein for dipeptide synthesis according to Claim 2.

20. The process according to Claim 19, wherein the microorganism transformed with DNA encoding the protein for dipeptide synthesis according to Claim 2 is a microorganism belonging to the genus Escherichia.

21. The process according to any of Claims 14 to 20, wherein the treated matter of the culture is concentrated culture, dried culture, cells obtained by centrifuging the culture, dried cells, freeze-dried cells, surfactant-treated cells, ultrasonic-treated cells, mechanically disrupted cells, solvent-treated cells, enzyme-treated cells, protein fractionation of the cells, immobilized cells, or an enzyme preparation obtainable from the cells by extraction.

22. The process according to any of Claims 13 to 21, wherein the one or more kinds of amino acids are L- or D-form of amino acids, glycine, β-alanine or derivatives thereof.

23. The process according to any of Claims 13 to 22, wherein the dipeptide is a dipeptide represented by formula (II):
R³ - R⁴ (II)
(wherein R³ and R⁴, which may be the same or different, each represent L- or D-form of amino acid, glycine, β-alanine or a derivative thereof).

24. The process according to Claim 22 or 23, wherein the L- or D-form of amino acid is an amino acid selected from the group consisting of alanine, glutamine, glutamic acid, valine, leucine, isoleucine, proline, phenylalanine, tryptophan, methionine, serine, threonine, cysteine, asparagine, tyrosine, lysine, arginine, histidine, aspartic acid, α-aminobutyric acid, azaserine, theanine, 4-hydroxyproline, 3-hydroxyproline, ornithine, citrulline and 6-diazo-5-oxo-norleucine.
